# EUROPEAN PATENT SPECIFICATION

(11) **EP 1 235 772 B1**
(45) Date of publication and mention of the grant of the patent: **19.01.2005**
(21) Application number: 00968627.0
(22) Date of filing: 03.10.2000
(51) Int. Cl.: C07C 17/156, C07C 17/154, C07C 17/152, C07C 21/06, C07C 17/25, B01J 27/06, B01J 27/10, B01J 27/125, C07C 1/30

(54) **A PROCESS FOR THE CONVERSION OF ETHYLENE TO VINYL CHLORIDE, AND NOVEL CATALYST COMPOSITIONS USEFUL FOR SUCH PROCESS**
VERFAHREN ZUR UMWANDLUNG VON ETHYLEN ZU VINYLCHLORID UND KATALYSATORZUSAMMENSETZUNGEN VERWENDBAR FÜR SOLCHES VERFAHREN
PROCEDE DE CONVERSION D'ETHYLENE EN CHLORURE DE VINYLE, ET COMPOSITIONS CATALYTIQUES UTILES DANS CE PROCEDE

(30) Priority: 22.11.1999 US 166897 P
(43) Date of publication of application: 04.09.2002
(73) Proprietor: Dow Global Technologies, Inc., Midland, Michigan 48674 (US)
(72) Inventor: JONES, Mark, E., Midland, MI 48642 (US); OLKEN, Michael, M., Midland, MI 48642 (US); HICKMAN, Daniel, A., Midland, MI 48642 (US)
(74) Representative: Sternagel, Fleischer, Godemeyer & Partner Patentanwälte
(86) International application number: PCT/US2000/027272
(87) International publication number: WO 2001/038273

(56) References cited:
- EP-A- 0 162 457
- EP-A- 0 501 757
- GB-A- 1 213 202

## Description

Vinyl chloride monomer (VCM) is used extensively as a monomer in the manufacture of polyvinyl chloride (PVC) resins, large volume, versatile plastic materials. This invention disclosed herein relates to a process and catalyst for the catalytic production of VCM from ethylene-containing streams. The process uses a novel catalyst allowing direct production of VCM in a single reactor system. Ethane may also, as a substantial further advantage, be included as a feedstock into this reactor system.

Presently, VCM is most commonly produced from ethylene and chlorine by first chlorinating ethylene to produce 1,2-dichloroethane. The 1,2-dichloroethane is then thermally dehydrochlorinated to yield VCM and a hydrogen chloride (HCl) by-product. The HCl produced in the dehydrochlorination reactor is typically captured and supplied to an oxychlorination reactor. The oxychlorination process catalytically converts ethylene, HCl and oxygen to 1,2-dichloroethane, which is also dehydrochlorinated to yield VCM. Consequently, the above process generally includes three separate reactor sections - a direct chlorination section, an oxychlorination section and a dehydrochlorination section. Plants operated in this manner introduce ethylene, chlorine and oxygen, and produce. substantially VCM and water. The complexity of the three reactor sections has led to a search for methods to produce VCM directly from hydrocarbon feedstocks in a single reactor section.

Further, ethylene is a capital-intensive material to produce, and the cost of ethylene generally is a significant factor in the total cost of producing VCM according to the above-described process. Precisely because of this last-described disadvantage of the conventional balanced technology, it has also long been sought to commercialize a process for producing VCM from ethane as a starting material.

A further disadvantage of the prior art for direct production of VCM common to both the ethane- and ethylene-based processes pertains to a less than desirable selectivity to VCM (often being less than 30 percent). This less than desirable selectivity to VCM is largely attributable to formation of byproducts during the oxychlorination reaction. Most of the by-products either derive from combustion products which are generated by oxidation of hydrocarbons such as ethane to form, mainly, CO and CO₂ (the combination of which will be referred to as COₓ), or the by-products are various chlorinated hydrocarbon derivatives (commonly, ethyl chloride, 1,1-dichloroethane, 1,2-dichloroethane, 1,1,2-trichloroethane, 1,1-dichloroethylene, *cis* - 1,2-dichloroethylene, *trans -* 1,2-dichloroethylene, trichloroethylene, and perchloroethylene). Formation of tri-, tetra-, penta- and hexachlorinated species is particularly undesirable due to their toxicity and physical properties. Prior art has proposed handling these byproducts primarily by means of either vent and disposal or by selectively separating and recycling some of the chlorinated by-products back to the oxychlorination reactor. Typically, the recycling requires numerous purification and conversion steps prior to utilizing the recycled products in the oxychlorination reactor. For example, the unsaturated chlorinated hydrocarbons are typically converted to saturated forms by a hydrogenation step.

GB-A-1,213,202 discloses a process for the production of vinyl chloride which comprises passing (a) ethane, ethylene or a mixture thereof, and (b) chlorine, hydrogen chloride or a mixture thereof, into a first reaction zone in which is a melt containing at least one chloride of a multivalent metal and the oxychloride of that metal to produce a gaseous effluent including vinyl chloride and 1,2-dichloroethane, withdrawing the gaseous effluent from the first reaction zone and separately recovering vinyl chloride and 1,2-dichloroethane as products therefrom; contacting the recovered 1,2-dichloroethane with a catalyst comprising a melt containing a chloride of said metal in a second reaction zone wherein 1,2-dichloroethane is dehydrochlorinated to produce an effluent containing vinyl chloride and separating vinyl chloride as product therefrom. Preferably copper catalysts are used.

EP-A-501 757 relates to a process for the production of a hydrocarbon derivative by the vapor phase reaction of a hydrocarbon with an oxygen-containing gas in the presence of a suitable catalyst in a reactor to produce a flammable gaseous product stream comprising the desired derivative, unreacted hydrocarbon, oxygen, carbon monoxide and carbon dioxide. Preferred examples of the hydrocarbon derivatives are vinyl chloride and ethylene dichloride.

EP-A-162 457 discloses a process for the manufacture of vinyl chloride from ethylene dichloride by catalyzed dehydrohalogenation of ethylene dichloride in the presence of oxygen and a catalyst comprising a rare earth metal chloride on a zeolite.

In a first aspect of the present invention, there is provided a composition of matter that is useful as a catalyst for producing vinyl chloride from ethylene. The composition is of the formula MOCl, wherein M is at least one rare earth element chosen from lanthanum, cerium, neodymium, praseodymium, dysprosium, samarium, yttrium, gadolinium, erbium, ytterbium, holmium, terbium, europium, thulium, lutetium, or mixtures thereof, with the proviso that, when cerium is present, at least one more rare earth element other than cerium is also present. A method for forming this composition comprises the following steps: (a) preparing a solution of a chloride salt of the rare earth element or elements in a solvent comprising either water, an alcohol, or mixtures thereof; (b) adding a nitrogen-containing base to cause the formation of a precipitate; and (c) collecting, drying and calcining the precipitate in order to form the MOCl composition.

In a second aspect of the present invention, an additional composition of matter is provided which is useful as a catalyst for producing vinyl chloride from ethylene. The composition is of the formula MCl₃, wherein M is at least one rare earth element from lanthanum, cerium, neodymium, praseodymium, dysprosium, samarium, yttrium, gadolinium, erbium, ytterbium, holmium, terbium, europium, thulium, lutetium, or mixtures thereof, with the proviso that, when cerium is present, at least one more rare earth element other than cerium is also present. A method for forming this composition comprises the following steps: (a) preparing a solution of a chloride salt of the rare earth element or elements in a solvent comprising either water, an alcohol, or mixtures thereof; (b) adding a nitrogen-containing base to cause the formation of a precipitate; (c) collecting, drying and calcining the precipitate; and (d) contacting the calcined precipitate with a chlorine source.

In a third aspect of the present invention a process for producing vinyl chloride from ethylene is provided comprising:
(a) combining reactants including ethylene, an oxygen source, and a chlorine source in a reactor containing a catalyst under conditions sufficient to produce a product stream comprising vinyl chloride and ethylene, the catalyst comprising a composition according to any of the above first and second aspects of the present invention, with the proviso that the catalyst is substantially free of iron and copper and with the further proviso that when cerium is present the catalyst further comprises at least one more rare earth element other than cerium.

According to a preferred embodiment of the process of the present invention ethylene is recycled from the product stream back to the reactor for carrying out the first step. Preferably, after drying according to methods known in the art, the hydrogen chloride produced in the product stream is also then recycled back for use in the first step. Carbon monoxide present in the product stream may also be recycled back to the first step of the process.

In contrast to the known processes, high VCM selectivity can be produced by the process of the present invention from an ethylene-containing feed, by using catalysts of the character described herein. Typically, VCM selectivity for the process is greater than 50 percent, based on C₂ converted. C₂ refers to ethylene fed to the reactor system as the sole hydrocarbon source or in combination with ethane. Preferably, VCM selectivity is greater than 60 percent, based on C₂ converted. More preferably, VCM selectivity is greater than 65 percent, based on C₂ converted, and most preferably, VCM selectivity for the process is greater than 70 percent, based on C₂ converted. One reason for the higher VCM selectivities is due to the fact that, at typical temperatures of operation for the process (which are generally lower than disclosed in comparative prior art processes for making VCM), the catalysts disclosed herein enable significantly reduced levels of the undesirable higher chlorinated species such as the tri-, tetra-, penta- and hexachlorinated species.

An additional advantage of this process is that it may employ ethane with the ethylene as a hydrocarbon source. Preferably, much of the ethane is oxidatively dehydrogenated to ethylene in the reactor. The catalyst and process of the present invention allow the recycle of part or all of the ethylene from the product stream directly back to the reactant stream. Any unreacted ethane present in the product stream can advantageously also be recycled back to the first step of the process. Optionally, other light gases, such as the products of combustion, can be contained in the recycled stream. When utilizing a co-feed of ethane, the process is preferably operated with an ethylene balance such that the total moles per minute (that is, "flux") of ethylene in the product stream is substantially equal to the total moles per minute of ethylene entering the reactor. In effect, the ethylene has the appearance of being continuously recycled without depletion while the ethane is substantially consumed in the reactor. A preferred mode of practicing the invention is thus for the recycle stream to become the sole source of ethylene for the first step and for ethane to provide the source of new C₂ hydrocarbon into the process.

The preferred chlorine and oxygen sources are gases. The most preferred oxygen source is oxygen. Desirable chlorine sources comprise hydrogen chloride, chlorine, chlorinated hydrocarbons containing labile chlorines, and mixtures thereof. Preferred chlorine sources which are considered "chlorinated hydrocarbons containing labile chlorines" include carbon tetrachloride, 1,2-dichloroethane, ethyl chloride, and mixtures thereof. Most preferably, at least some measure of chlorine gas (Cl₂) is continuously present in the reactant stream. It has been determined in this regard that when Cl₂ is employed in the reactant stream as a chlorine source, for any given set of conditions the amount of combustion products (COₓ) can be reduced compared to where Cl₂ is not so used. Alternatively, where another chlorine source, for example, hydrogen chloride (including hydrogen chloride recovered from the product stream and recycled), is contemplated to be used as the sole chlorine source in normal operations, then Cl₂ will be supplied to the catalyst both initially and after an interruption in the process before bringing the process fully back on-line, on the additional finding that after treatment (or pretreatment) with Cl₂ the catalyst's tendency to make these products of combustion can be reduced substantially, compared to the circumstance wherein Cl₂ has not been used to treat or condition the catalyst.

In light of the disclosure herein, those of skill in the art are capable of varying conditions in the reactor in order for the conditions to be sufficient for producing a product stream comprising vinyl chloride, ethylene, and hydrogen chloride. Conditions which are typically varied by those skilled in the art include: the molar feed ratios of the reactants; temperature; pressure; and space time. Preferably, the reactor is maintained between a temperature of greater than 350 degrees Celsius, more preferably greater than 375 degrees Celsius and a temperature less than 500 degrees Celsius, more preferably less than 450 degrees Celsius. Typically, the reactor is maintained between ambient pressure and 3.5 megapascals (MPa), gauge (500 pounds per square inch, gauge (psig)). Operation at pressure allows considerable flexibility to the down-stream processing operations, since higher pressure provides a driving force for the movement of materials into and through separation unit operations. Preferably, the operating pressure is between ambient and 2.1 MPa, gauge (300 psig), and most preferably between ambient and 1.1 MPa, gauge (150 psig). The process can be carried out in either a fixed bed or fluidized bed mode, though a fluidized process is preferred.

Another aspect pertains to the catalyst utilized for the process of this invention. While the aforementioned process is the primary focus for the catalyst that is herein disclosed, the catalyst does have additional utilities, for example, as a catalyst precursor, as a regenerable absorbent, as a catalyst support, and as a catalyst for other processes. As illustrative, rare earth oxychlorides can be used as regenerable bases by exposing them to HCl, wherein they are converted to their respective rare earth chlorides, liberating water. Exposure of rare earth chlorides to water result in conversion back to rare earth oxychlorides, liberating HCl. It is noteworthy that particles and pellets of rare earth oxychlorides do not undergo gross changes in shape or dimension upon chlorination. In contrast, pure oxides of the rare earths can undergo gross changes upon chlorination which cause severe fracturing of prepared particles. Rare earth chlorides also react with methanol to yield methyl chloride. Therefore, the catalyst can be used in catalytic processes for production of methyl chloride free of HCl.

The catalyst can also be useful for ethane dehydrogenation since contacting a stream of ethane, oxygen and a chlorine source such as HCl with the catalyst results in the production of a stream comprising predominantly ethylene and HCl. In addition, contacting the catalyst with a stream containing one or more of ethyl chloride, 1,2-dichloroethane and 1,1,2-trichloroethane results in the hydrodechlorination of these materials to yield HCl and a respective, corresponding unsaturated hydrocarbon or chlorohydrocarbon. Furthermore, when copper salts are contacted with the catalyst (either by their presence in solution during the precipitation or by introduction of copper containing solutions to the calcined catalyst), treating the catalyst with HCl yields a catalyst which is useful for the oxychlorination of ethylene to 1,2-dichioroethane. The catalysts are particularly desirable due to their ability to run at higher temperatures without increased production of COₓ.

As described previously, the catalyst of this invention comprises at least one rare earth material. The rare earths are a group of 17 elements consisting of scandium (atomic number 21), yttrium (atomic number 39) and the lanthanides (atomic numbers 57-71) [James B. Hedrick, U.S. Geological Survey - Minerals Information - 1997, "Rare-Earth Metals"]. The catalyst can be provided as porous, bulk material. Preferred rare earth materials are those based on lanthanum, cerium, neodymium, praseodymium, dysprosium, samarium, yttrium, gadolinium, erbium, ytterbium, holmium, terbium, europium, thulium, and lutetium. Most preferred rare earth materials for use in the aforementioned VCM process are based on those rare earth elements which are typically considered as being single valency materials. Catalytic performance of multi-valency materials appears to be less desirable than those that are single valency. For example, cerium is known to be an oxidation-reduction catalyst having the ability to access both the 3⁺ and 4⁺ stable oxidation states. This is one reason why, if the rare earth material is based on cerium, the catalyst of this invention further comprises at least one more rare earth element other than cerium. Preferably, if one of the rare earths employed in the catalyst is cerium, the cerium is provided in a molar ratio that is less than the total amount of other rare earths present in the catalyst. More preferably, however, substantially no cerium is present in the catalyst. By "substantially no cerium" it is meant that any cerium is in an amount less than 33 atom percent of the rare earth components, preferably less than 20 atom percent, and most preferably less than 10 atom percent.

The rare earth material for the catalyst of this invention is more preferably based upon lanthanum, neodymium, praseodymium or mixtures of these. Most preferably, at least one of the rare earths used in the catalyst is lanthanum. Furthermore, for the ethylene-containing feed to VCM process of this invention, the catalyst is substantially free of iron and copper. In general, the presence of materials that are capable of oxidation-reduction (redox) is undesirable for the catalyst. It is preferable for the catalyst to also be substantially free of other transition metals that have more than one stable oxidation state. For example, manganese is another transition metal that is preferably excluded from the catalyst. By "substantially free" it is meant that the atom ratio of rare earth element to redox metal in the catalyst is greater than 1, preferably greater than 10, more preferably greater than 15, and most preferably greater than 50.

It may also be advantageous to include other elements within the catalyst in the porous, bulk material forms. For example, preferable elemental additives include alkaline earths, boron, phosphorous, sulfur, silicon, germanium, titanium, zirconium, hafnium, aluminum, and combinations thereof. These elements can be present to after the catalytic performance of the composition or to improve the mechanical properties (for example attrition-resistance) of the material.

Prior to combining the ethylene-containing feed, oxygen source, and chlorine source in the reactor for the VCM process embodiment of this invention, it is preferable for the catalyst composition to comprise a salt of at least one rare earth element with the proviso that the catalyst is substantially free of iron and copper and with the further proviso that when cerium is employed the catalyst further comprises at least one more rare earth element other than cerium. The salt of at least one rare earth element is preferably selected from rare earth oxychlorides, rare earth chlorides, rare earth oxides, and combinations thereof, with the proviso that the catalyst is substantially free of iron and copper and with the further proviso that when cerium is used the catalyst further comprises at least one more rare earth element other than cerium. More preferably, the salt comprises a rare earth oxychloride of the formula MOCl, wherein M is at least one rare earth element chosen from lanthanum, cerium, neodymium, praseodymium, dysprosium, samarium, yttrium, gadolinium, erbium, ytterbium, holmium, terbium, europium, thulium, lutetium, or mixtures thereof, with.the proviso that, when cerium is present, at least one more rare earth element other than cerium is also present. Most preferably, the salt is a porous, bulk lanthanum oxychloride (LaOCl) material. As has been mentioned, this material beneficially does not undergo gross changes (for example, fracturing) when chlorinated in situ in this process, and provides the further beneficial property of water solubility in the context of this process after a period of use (LaOCl is initially water-insoluble), so that should spent catalyst need to be removed from a fluidized bed, fixed bed reactor or other process equipment or vessels, this can be done without hydroblasting or conventional labor-intensive mechanical techniques by simply flushing the spent catalyst from the reactor in question with water.

When the salt is a rare earth oxychloride (MOCl), it has a BET surface area of at least 12 m²/g, preferably at least 15 m²/g, more preferably at least 20 m²/g, and most preferably at least 30 m²/g. Generally, the BET surface area is less than 200 m²/g. For these above measurements, the nitrogen adsorption isotherm was measured at 77K and the surface area was calculated from the isotherm data utilizing the BET method (Brunauer, S., Emmett, P.H., and Teller, E., J. Am. Chem. Soc., 60, 309 (1938)). In addition, it is noted that the MOCI phases possess characteristic powder X-Ray Diffraction (XRD) patterns that are distinct from the MCl₃ phases.

It is also possible, as indicated in several instances previously, to have mixtures of the rare earths ("M") within the MOCI composition. For example, M can be a mixture of at least two rare earths selected from lanthanum, cerium, neodymium, praseodymium, dysprosium, samarium, yttrium, gadolinium, erbium, ytterbium, holmium, terbium, europium, thulium and lutetium. Similarly, it is also possible to have mixtures of different MOCI compositions wherein M is different as between each composition of the MOCl's in the mixture.

Once the ethylene-containing feed, oxygen source, and chlorine source are combined in the reactor, a catalyst is formed *in situ* from the salt of at least one rare earth element. Although this characterization should not limit the composition or process of this invention in any way, it is believed that the *in situ* formed catalyst comprises a chloride of the rare earth component. An example of such a chloride is MCl₃, wherein M is a rare earth component selected from lanthanum, cerium, neodymium, praseodymium, dysprosium, samarium, yttrium, gadolinium, erbium, ytterbium, holmium, terbium, europium, thulium, lutetium and mixtures thereof, with the proviso that when cerium is present the catalyst further comprises at least one more rare earth element other than cerium. When the salt is a rare earth chloride (MCl₃), it has a BET surface area of at least 5 m²/g, preferably at least 10 m²/g, more preferably at least 15 m²/g, more preferably at least 20 m²/g, and most preferably at least 30 m²/g.

Oxychlorination is conventionally referenced as the oxidative addition of two chlorine atoms to ethylene from HCl or other reduced chlorine source. Catalysts capable of performing this chemistry have been classified as modified Deacon catalysts [Olah, G. A., Molnar, A., Hydrocarbon Chemistry, John Wiley & Sons (New York, 1995), pg 226]. Deacon chemistry refers to the Deacon reaction, the oxidation of HCl to yield elemental chlorine and water.

Without being limiting of the present invention as claimed hereafter, in contrast to oxychlorination, the preferred process and catalyst described above are considered as utilizing oxydehydro-chlorination in converting ethane-containing and ethylene-containing streams to VCM at high selectivity. Oxydehydro-chlorination is the conversion of a hydrocarbon (using oxygen and a chlorine source) to a chlorinated hydrocarbon wherein the carbons either maintain their initial valence or have their valency reduced (i.e., sp³ carbons remain sp³ or are converted to sp², and sp² carbons remain sp² or are converted to sp). This differs from the conventional definition of oxychlorination, whereby ethylene is converted to 1,2-dichloroethane with a net increase in carbon valency (i.e., sp² carbons are converted to sp³ carbons).

In light of the disclosure herein, those of skill in the art will undoubtedly recognize alternative methods for preparing the compositions of this invention. A method currently felt to be preferable, however, for forming the composition comprising the rare earth oxychloride (MOCl) comprises the following steps: (a) preparing a solution of a chloride salt of the rare earth element or elements in a solvent comprising either water, an alcohol, or mixtures thereof; (b) adding a nitrogen-containing base to cause the formation of a precipitate; and (c) collecting, drying and calcining the precipitate in order to form the MOCl material. Typically, the nitrogen-containing base is selected from ammonium hydroxide, alkyl amine, aryl amine, arylalkyl amine, alkyl ammonium hydroxide, aryl ammonium hydroxide, arylalkyl ammonium hydroxide, and mixtures thereof. The nitrogen-containing base may also be provided as a mixture of a nitrogen-containing base with other bases that do not contain nitrogen. Preferably, the nitrogen-containing base is tetra-alkyl ammonium hydroxide. The solvent in Step (a) is preferably water. Drying of the catalytically-useful composition can be done in any manner, including by spray drying, drying in a purged oven and other known methods. For the presently-preferred fluidized bed mode of operation, a spray-dried catalyst is preferred.

A method currently felt to be preferable for forming the catalyst composition comprising the rare earth chloride (MCl₃) comprises the following steps: (a) preparing a solution of a chloride salt of the rare earth element or elements in a solvent comprising either water, an alcohol, or mixtures thereof; (b) adding a nitrogen-containing base to cause the formation of a precipitate; (c) collecting, drying and calcining the precipitate; and (d) contacting the calcined precipitate with a chlorine source. For example, one application of this method (using La to illustrate) would be to precipitate LaCl₃ solution with a nitrogen containing base, dry it, add it to the reactor, heat it to 400°C in the reactor to perform the calcination, and then contact the calcined precipitate with a chlorine source to form the catalyst composition *in situ* in the reactor.

### Examples

The invention will be further clarified by a consideration of the following examples, which are intended to be purely exemplary.

### Reference Example 1

To demonstrate the production of vinyl chloride from a stream comprising ethylene. a porous, refractory composition comprising lanthanum was prepared. A solution of LaCl₃ in water was prepared by dissolving one part of commercially available hydrated lanthanum chloride (obtained from J.T. Baker Chemical Company) in 8 parts of deionized water. Dropwise addition with stirring of ammonium hydroxide (obtained from Fisher Scientific, certified ACS specification) to neutral pH (by universal test paper) caused the formation of a gel. The mixture was centrifuged, and the solution decanted away from the solid. Approximately 150 ml of deionized water was added and the gel was stirred vigorously to disperse the solid. The resulting solution was centrifuged and the solution decanted away. This washing step was repeated two additional times. The collected, washed gel was dried for two hours at 120 degrees Celsius and subsequently calcined at 550 deg. C. for four hours in air. The resulting solid was crushed and sieved to yield particles suitable for additional testing. This procedure produced a solid matching the X-ray powder diffraction pattern of LaOCl.

The particles were placed in a pure nickel (alloy 200) reactor. The reactor was configured such that ethylene, ethane, HCl, O₂ and inert gas (He and Ar mixture) could be fed to the reactor. The function of the argon was as an internal standard for the analysis of the reactor feed and effluent by gas chromatography. Space time is calculated as the volume of catalyst divided by the flow rate at standard conditions. Feed rates are molar ratios. The reactor system was immediately fed an ethane-containing stream with the stoichiometry of one ethane, one HCl and one oxygen. This provides balanced stoichiometry for the production of VCM from ethylene.

Table 1 below sets forth the results of reactor testing using this composition.

Column 1 of Table I shows the high selectivity to vinyl chloride when the catalyst system is fed ethylene under oxidizing conditions in the presence of HCl. The composition contains helium in order to mimic a reactor operated with air as the oxidant gas.

Column 2 of Table 1 shows the high selectivity to vinyl chloride when the catalyst system is fed ethylene under oxidizing conditions in the presence of HCl. The composition is now fuel rich to avoid limitations imposed by flammability and contains no helium.

Column 3 of Table I shows the high selectivity to vinyl chloride and ethylene when the catalyst system is fed ethane under oxidizing conditions in the presence of HCl. The composition mimics a reactor operated with air as the oxidant gas. There is no ethylene present in the feed. The ethylene present in the reactor is the product of the partial oxidation of ethane.

Column 4 of Table 1 shows the result when both ethane and ethylene are fed. The reactor is operated in such a way as to insure that the amount of ethylene entering the reactor and exiting the reactor are equal. Operated in this fashion, the ethylene gives the appearance of an inert diluent, and only ethane is being converted. The results show a high yield of vinyl chloride and 1,2-dichloroethane. Argon is used as an internal standard to insure that the ethylene flux entering the reactor and the ethylene flux exiting the reactor are equal. The ratio of the ethylene to argon integrated chromatographic peak is identical for the reactor feed and product stream. In this way the recycle of ethylene is simulated within the reactor device.

**Table 1**

| Feed Mole Ratios | | | | |
|---|---|---|---|---|
| C₂H₄ | 2 | 3.7 | 0 | 3 |
| C₂H₆ | 0 | 0 | 1 | 2 |
| HCl | 2 | 2 | 1 | 2.5 |
| O₂ | 1 | 1 | 1 | 1 |
| Inerts | 6.8 | 0 | 4 | 0 |
| T (deg. C) | 401 | 400 | 401 | 419 |
| Space time (s) | 12.3 | 5.0 | 21.8 | 12.4 |
| | | | | |
| O₂ conv. (pct) | 47.3 | 53.7 | 54.8 | 93.9 |

| Selectivities (Percent) | | | | |
|---|---|---|---|---|
| C₂H₄ | -- | -- | 44.7 | -- |
| C₂H₄Cl₂ | 10.7 | 14.0 | 0.1 | 12.8 |
| VCM | 76.6 | 78.1 | 34.5 | 68.5 |

### Reference Example 2

To further demonstrate the utility of the composition, ethylene is oxidatively converted to vinyl chloride using a variety of chlorine sources. A solution of LaCl₃ in water was prepared by dissolving one part of commercially available hydrated lanthanum chloride (purchased from Avocado Research Chemicals Ltd.) in 6.6 parts of deionized water. Rapid addition with stirring of 6 M ammonium hydroxide in water (diluted certified ACS reagent, obtained from Fisher Scientific) caused the formation of a gel. The mixture was filtered to collect the solid. The collected gel was dried at 120 deg C prior to calcination at 550 deg C for four hours in air. The resulting solid was crushed and sieved. The sieved particles were placed in a pure nickel (alloy 200) reactor. The reactor was configured such that ethylene, HCl, oxygen, 1,2-dichloroethane, carbon tetrachloride and helium could be fed to the reactor. Space time is calculated as the volume of catalyst divided by the flow rate at standard temperature and pressure. Feed rates are molar ratios. The composition was heated to 400 deg C and treated with a 1:1:3 HCl:O₂:He mixture for 2 hours prior to the start of operation.

The composition formed was operated to produce vinyl chloride by feeding ethylene, chlorine source and oxygen at 400 deg C. The following table shows data obtained between 82 and 163 hours on stream using different chlorine sources. Chlorine is supplied as HCl, carbon tetrachloride and 1,2-dichloroethane. VCM signifies vinyl chloride. Space time is calculated as the volume of catalyst divided by the flow rate at standard temperature and pressure. The reactors are operated with the reactor exit at ambient pressure. Both ethylene and 1,2-dichloroethane are termed to be C₂ species.

**Table 2**

| Feed mole ratios | | | | |
|---|---|---|---|---|
| C₂H₄ | 2.0 | 2.0 | 2.0 | 2.0 |
| C₂H₆ | 0.0 | 0.0 | 0.0 | 0.0 |
| CCl₄ | 0.5 | 0.5 | 0.0 | 0.0 |
| C₂H₄Cl₂ | 0.0 | 0.0 | 1.8 | 0.0 |
| HCl | 0.0 | 0.0 | 0.0 | 1.9 |
| O₂ | 1.0 | 1.0 | 1.0 | 1.0 |
| He+Ar | 8.9 | 9.0 | 8.9 | 6.7 |
| T (deg C) | 400 | 399 | 401 | 400 |
| Space time (s) | 8.0 | 4.0 | 8.6 | 4.9 |

| Fractional conversions (Percent) | | | | |
|---|---|---|---|---|
| C₂H₄ | 40.4 | 27.0 | 18.7 | 20.1 |
| C₂H₆ | 0.0 | 0.0 | 0.0 | 0.0 |
| CCl₄ | 94.8 | 78.4 | 0.0 | 0.0 |
| C₂H₄Cl₂ | 0.0 | 0.0 | 98.3 | 0.0 |
| HCl | 0.0 | 0.0 | 0.0 | 44.7 |
| O₂ | 68.8 | 42.0 | 55.2 | 37.8 |

| Selectivities based on moles of C₂ converted | | | | |
|---|---|---|---|---|
| VCM | 59.6 | 56.4 | 86.0 | 78.5 |
| C₂H₄Cl₂ | 14.8 | 30.7 | 0.0 | 2.2 |
| C₂H₅Cl | 0.6 | 0.4 | 0.2 | 1.6 |

These data show that a variety of chlorine sources can be used in the oxidative production of vinyl. The use of carbon tetrachloride, 1,2-dichloroethane and HCl all produce vinyl chloride as the dominant product.

### Reference Example 3

A solution of LaCl₃ in water was prepared by dissolving one part of commercially available hydrated lanthanum chloride (purchased from Avocado Research Chemicals Ltd.) in 6.67 parts of deionized water. Rapid addition with stirring of 6 M ammonium hydroxide in water (diluted certified ACS reagent, obtained from Fisher Scientific) caused the formation of a gel and yielded a final pH of 8.85. The mixture was filtered to collect the solid. The collected material was calcined in air at 550 deg C for four hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor.

Table 3 shows data wherein the reactor feeds were adjusted such that the flux of ethylene (motes/minute) entering the reactor and the flux of ethylene exiting the reactor were substantially equal. Reactor feeds were similarly adjusted such that the fluxes of HCl entering and exiting the reactor were substantially equal. Oxygen conversion was set at slightly less than complete conversion to enable the monitoring of catalyst activity. Operated in this manner, the consumed feeds are ethane, oxygen, and chlorine. Both ethylene and HCl give the appearance of neither being created nor consumed. Space time is calculated as the volume of catalyst divided by the flow rate at standard temperature and pressure. The example further illustrates the use of chlorine gas as a chlorine source in the production of vinyl chloride.

**Table 3**

| Feed mole ratios | |
|---|---|
| C₂H₄ | 2.1 |
| C₂H₆ | 4.5 |
| Cl₂ | 0.5 |
| HCl | 2.4 |
| O₂ | 1.0 |
| He+Ar | 7.4 |
| T (°C) | 400 |
| Space time (s) | 9.4 |

| Fractional conversions (Pct.) | |
|---|---|
| C₂H₄ | 1.8 |
| C₂H₆ | 27.3 |
| Cl₂ | 99.8 |
| HCl | -1.4 |
| O₂ | 96.4 |

| Selectivities (Pct) | |
|---|---|
| VCM | 79.0 |
| C₂H₄Cl₂ | 7.2 |
| C₂H₅Cl | 1.7 |
| COₓ | 5.1 |
| C₂H₄ | 0.5 |

In common with all examples herein, VCM signifies vinyl chloride. C₂H₄Cl₂ is solely 1,2-dichloroethane. COₓ is the combination of CO and CO₂.

### Reference Example 4

The catalyst composition prepared in Example 1 was operated to show the effect of temperature on catalyst performance. The results are shown in Table 4.

**Table 4:**

| Temperature Effects on Lanthanum Composition | | | |
|---|---|---|---|
| Feed mole ratios | | | |
| C₂H₄ | 1.9 | 1.9 | 1.9 |
| C₂H₆ | 0.0 | 0.0 | 0.0 |
| Cl₂ | 0.0 | 0.0 | 0.0 |
| HCl | 1.9 | 1.9 | 1.5 |
| O₂ | 1.0 | 1.0 | 1.0 |
| He+Ar | 6.6 | 6.6 | 7.1 |
| T (°C) | 349 | 399 | 450 |
| Space time (s) | 4.9 | 9.7 | 9.6 |

| Fractional conversions (Pct) | | | |
|---|---|---|---|
| C₂H₄ | 8.2 | 33.0 | 35.2 |
| C₂H₆ | 0.0 | 0.0 | 0.0 |
| Cl₂ | | | |
| HCl | 7.5 | 36.0 | 46.5 |
| O₂ | 8.8 | 49.2 | 57.1 |

| Selectivities (Pct) | | | |
|---|---|---|---|
| VCM | 67.7 | 87.4 | 79.8 |
| C₂H₄Cl₂ | 2.5 | 0.2 | 0.8 |
| C₂H₅Cl | 28.1 | 1.3 | 0.4 |
| COₓ | 1.6 | 0.9 | 8.9 |

These data show that the ability of the composition to produce vinyl chloride is little changed by increasing temperature. Lower temperature decreases rates but selectivity is only altered in a minor way.

### Example 5 through Example 12

Example 5 through Example 12 illustrate the preparation of numerous rare earth compositions, each containing only one rare earth material. Data illustrating the performance of these compositions are set forth in Table 5.

### Example 5

A solution of LaCl₃ in water was prepared by dissolving one part of commercially available hydrated lanthanum chloride (purchased from Aldrich Chemical Company) in 6.67 parts of deionized water. Rapid addition with stirring of 6 M ammonium hydroxide in water (diluted certified ACS reagent, obtained from Fisher Scientific) caused the formation of a gel. The mixture was centrifuged to collect the solid. Solution was decanted away from the gel and discarded. The gel was resuspended in 6.66 parts of deionized water. Centrifuging allowed collection of the gel. The collected gel was dried at 120 deg C prior to calcination at 550 deg C for four hours in air. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. Powder x-ray diffraction shows the material to be LaOCl. The BET surface area is measured to be 42.06 m²/g. The specific performance data for this example are set forth below in Table 5.

### Example 6

A solution of NdCl₃ in water was prepared by dissolving one part of commercially available hydrated neodymium chloride (Alfa Aesar) in 6.67 parts of deionized water. Rapid addition with stirring of 6 M ammonium hydroxide in water (diluted certified ACS reagent, obtained from Fisher Scientific) caused the formation of a gel. The mixture was filtered to collect the solid. The collected gel was dried at 120 deg C prior to calcination in air at 550 deg C for four hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. Powder x-ray diffraction shows the material to be NdOCl. The BET surface area is measured to be 22.71 m²/g. The specific performance data for this example are set forth below in Table 5.

### Example 7

A solution of PrCl₃ in water was prepared by dissolving one part of commercially available hydrated praseodymium chloride (Alfa Aesar) in 6.67 parts of deionized water. Rapid addition with stirring of 6 M ammonium hydroxide in water (diluted certified ACS reagent, obtained from Fisher Scientific) caused the formation of a gel. The mixture was filtered to collect the solid. The collected gel was dried at 120 deg C prior to calcination in air at 550 deg C for four hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. Powder x-ray diffraction shows the material to be PrOCl.
The BET surface area is measured to be 21.37 m²/g. The specific performance data for this example are set forth below in Table 5.

### Example 8

A solution of SmCl₃ in water was prepared by dissolving one part of commercially available hydrated samarium chloride (Alfa Aesar) in 6.67 parts of deionized water.
Rapid addition with stirring of 6 M ammonium hydroxide in water (diluted certified ACS reagent, obtained from Fisher Scientific) caused the formation of a gel. The mixture was filtered to collect the solid. The collected gel was dried at 120 deg C prior to calcination at 500 deg C for four hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. Powder x-ray diffraction shows the material to be SmOCl. The BET surface area is measured to be 30.09 m²/g. The specific performance data for this example are set forth below in Table 5.

### Example 9

A solution of HoCl₃ in water was prepared by dissolving one part of commercially available hydrated holmium chloride (Alfa Aesar) in 6.67 parts of deionized water. Rapid addition with stirring of 6 M ammonium hydroxide in water (diluted certified ACS reagent, obtained from Fisher Scientific) caused the formation of a gel. The mixture was filtered to collect the solid. The collected gel was dried at 120 deg C prior to calcination at 500 deg C for four hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. The BET surface area is measured to be 20.92 m²/g. The specific performance data for this example are set forth below in Table 5.

### Example 10

A solution of ErCl₃ in water was prepared by dissolving one part of commercially available hydrated erbium chloride (Alfa Aesar) in 6.67 parts of deionized water. Rapid addition with stirring of 6 M ammonium hydroxide in water (diluted certified ACS reagent, obtained from Fisher Scientific) caused the formation of a gel. The mixture was filtered to collect the solid. The collected gel was dried at 120 deg C prior to calcination at 500 deg C for four hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. The BET surface area is measured to be 19.80 m²/g. The specific performance data for this example are set forth below in Table 5.

### Example 11

A solution of YbCl₃ in water was prepared by dissolving one part of commercially available hydrated ytterbium chloride (Alfa Aesar) in 6.67 parts of deionized water.
Rapid addition with stirring of 6 M ammonium hydroxide in water (diluted certified ACS reagent, obtained from Fisher Scientific) caused the formation of a gel. The mixture was filtered to collect the solid. The collected gel was dried at 120 deg C prior to calcination at 500 deg C for four hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. The BET surface area is measured to be 2.23 m²/g. The specific performance data for this example are set forth below in Table 5.

### Example 12

A solution of YCl₃ in water was prepared by dissolving one part of commercially available hydrated yttrium chloride (Alfa Aesar) in 6.67 parts of deionized water. Rapid addition with stirring of 6 M ammonium hydroxide in water (diluted certified ACS reagent, obtained from Fisher Scientific) caused the formation of a gel. The mixture was filtered to collect the solid. The collected gel was dried at 120 deg C prior to calcination at 500 deg C for four hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. The BET surface area is measured to be 29.72 m²/g. The specific performance data for this example are set forth below in Table 5.

**Table 5:**

| Rare Earth Oxychloride Compositions Operated to Produce Vinyl Chloride | | | | | | | | |
|---|---|---|---|---|---|---|---|---|
| Example | 5 | 6 | 7 | 8 | 9 | 10 | 11 | 12 |
| Feed mole ratios | | | | | | | | |
| C₂H₄ | 3.6 | 4.2 | 3.7 | 3.6 | 3.6 | 3.6 | 4.2 | 3.6 |
| HCl | 2.0 | 2.3 | 2.0 | 2.0 | 2.0 | 2.0 | 2.3 | 2.0 |
| O₂ | 1.0 | 1.0 | 1.0 | 1.0 | 1.0 | 1.0 | 1.0 | 1.0 |
| He+Ar | 0.2 | 0.2 | 0.2 | 0.2 | 0.2 | 0.2 | 0.2 | 0.2 |
| T (deg C) | 399 | 403 | 401 | 400 | 400 | 400 | 400 | 399 |
| Space time (s) | 8.7 | 21.3 | 11.4 | 17.6 | 17.7 | 22.8 | 23.1 | 21.3 |

| Fractional conversions (Percent) | | | | | | | | |
|---|---|---|---|---|---|---|---|---|
| C₂H₄ | 23.7 | 13.2 | 22.8 | 14.7 | 12.7 | 15.4 | 3.3 | 13.8 |
| HCl | 47.6 | 24.9 | 40.9 | 20.8 | 15.9 | 22.4 | 5.0 | 19.8 |
| O₂ | 58.8 | 59.4 | 55.0 | 53.4 | 48.1 | 48.8 | 21.2 | 47.8 |

| Selectivities (Percent) | | | | | | | | |
|---|---|---|---|---|---|---|---|---|
| VCM | 75.3 | 74.4 | 74.2 | 61.0 | 33.3 | 44.0 | 6.1 | 35.0 |
| C₂H₄Cl₂ | 11.3 | 2.9 | 6.1 | 2.9 | 14.5 | 17.5 | 8.8 | 18.8 |
| C₂H₅Cl | 3.5 | 6.9 | 4.4 | 10.6 | 16.8 | 12.8 | 37.0 | 16.5 |
| COₓ | 4.8 | 11.8 | 9.7 | 22.4 | 33.8 | 23.1 | 26.4 | 27.5 |

These data show the utility of bulk rare earth containing compositions for the conversion of ethylene containing streams to vinyl chloride.

### Example 13 through Example 17

Example 13 through Example 17 illustrate the preparation of numerous rare earth compositions, each containing a mixture of rare earth materials. Data illustrating the performance of these data are set forth in Table 6.

### Example 13

A solution of LaCl₃ and NdCl₃ in water was prepared by dissolving one part of commercially available hydrated lanthanum chloride (purchased from Spectrum Quality Products) and 0.67 parts of commercially available hydrated neodymium chloride (Alfa Aesar) in 13.33 parts of deionized water. Rapid addition with stirring of 6 M ammonium hydroxide in water (diluted certified ACS reagent, obtained from Fisher Scientific) caused the formation of a gel. The final pH was measured as 8.96. The mixture was centrifuged to collect the solid. Solution was decanted away from the gel and discarded. The collected gel was dried at 80 deg C prior to calcination in air at 550 deg C for four hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. The BET surface area is measured to be 21.40 m²/g. The specific performance data for this example are set forth below in Table 6.

### Example 14

A solution of LaCl₃ and SmCl₃ in water was prepared by dissolving one part of commercially available hydrated lanthanum chloride (purchased from Spectrum Quality Products) and 0.67 parts of commercially available hydrated samarium chloride (Alfa Aesar) in 13.33 parts of deionized water. Rapid addition with stirring of 6 M ammonium hydroxide in water (diluted certified ACS reagent, obtained from Fisher Scientific) caused the formation of a gel. The final pH was measured as 8.96. The mixture was centrifuged to collect the solid. Solution was decanted away from the gel and discarded. The collected gel was dried at 80 deg C prior to calcination in air at 550 deg C for four hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. The BET surface area is measured to be 21.01 m²/g. The specific performance data for this example are set forth below in Table 6.

### Example 15

A solution of LaCl₃ and YCl₃ in water was prepared by dissolving one part of commercially available hydrated lanthanum chloride (purchased from Spectrum Quality Products) and 0.52 parts of commercially available hydrated yttrium chloride (Alfa Aesar) in 13.33 parts of deionized water. Rapid addition with stirring of 6 M ammonium hydroxide in water (diluted certified ACS reagent, obtained from Fisher Scientific) caused the formation of a gel. The final pH was measured as 8.96. The mixture was centrifuged to collect the solid. Solution was decanted away from the gel and discarded. The collected gel was dried at 80 deg C prior to calcination in air at 550 deg C for four hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. The BET surface area is measured to be 20.98 m²/g. The specific performance data for this example are set forth below in Table 6.

### Example 16

A solution of LaCl₃ and HoCl₃ in water was prepared by dissolving one part of commercially available hydrated lanthanum chloride (purchased from Spectrum Quality Products) and one part of commercially available hydrated holmium chloride (Alfa Aesar) in 13.33 parts of deionized Water. Rapid addition with stirring of 6 M ammonium hydroxide in water (diluted certified ACS reagent, obtained from Fisher Scientific) caused the formation of a gel. The final pH was measured as 8.64. The mixture was centrifuged to collect the solid. Solution was decanted away from the gel and discarded. The collected gel was dried at 80 deg C prior to calcination in air at 550 deg C for four hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. The BET surface area is measured to be 19.68 m²/g. The specific performance data for this example are set forth below in Table 6.

### Example 17

A solution of LaCl₃ and HoCl₃ in water was prepared by dissolving one part of commercially available hydrated lanthanum chloride (purchased from Spectrum Quality Products) and 0.75 parts of commercially available hydrated ytterbium chloride (Alfa Aesar) in 13.33 parts of deionized water. Rapid addition with stirring of 6 M ammonium hydroxide in water (diluted certified ACS reagent, obtained from Fisher Scientific) caused the formation of a gel. The final pH was measured as 9.10. The mixture was centrifuged to collect the solid. Solution was decanted away from the gel and discarded. The collected gel was dried at 80 deg C prior to calcination in air at 550 deg C for four hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. The BET surface area is measured to be 20.98 m²/g. The specific performance data for this example are set forth below in Table 6.

**Table 6:**

| Performance of Compositions Containing Two Rare earth materials | | | | | |
|---|---|---|---|---|---|
| Example | 13 | 14 | 15 | 16 | 17 |
| Feed mole ratios | | | | | |
| C₂H₄ | 3.7 | 3.6 | 3.6 | 3.6 | 3.6 |
| HCl | 2.0 | 2.0 | 2.0 | 2.0 | 2.0 |
| O₂ | 1.0 | 1.0 | 1.0 | 1.0 | 1.0 |
| He+Ar | 0.2 | 0.2 | 0.2 | 0.2 | 0.2 |
| T (°C) | 401 | 401 | 400 | 399 | 400 |
| Space time (s) | 3.7 | 15.7 | 13.7 | 16.9 | 20.6 |

| Fractional conversions (Percent) | | | | | |
|---|---|---|---|---|---|
| C₂H₄ | 16.8% | 11.3 | 12.5 | 12.4 | 9.2 |
| HCl | 36.0 | 13.1 | 18.1 | 11.9 | 15.9 |
| O₂ | 45.9 | 47.2 | 52.2 | 47.1 | 38.7 |

| Selectivities (Percent) | | | | | |
|---|---|---|---|---|---|
| VCM | 75.8 | 51.0 | 51.4 | 28.9 | 11.1 |
| C₂H₄Cl₂ | 9.7 | 7.5 | 12.4 | 14.5 | 20.6 |
| C₂H₅Cl | 4.1 | 11.8 | 8.9 | 17.0 | 23.8 |
| COₓ | 6.9 | 27.5 | 25.8 | 38.9 | 43.8 |

These data further show the utility of bulk rare earth containing compositions containing mixtures of the rare earth materials for the conversion of ethylene containing streams to vinyl chloride.

### Example 18 through Example 25

Example 18 through Example 25 are compositions containing rare earth materials with other additives present.

### Reference Example 18

A solution of LaCl₃ in water was prepared by dissolving one part of commercially available hydrated lanthanum chloride (purchased from Aldrich Chemical Company) in 6.67 parts of deionized water. 0.48 parts of ammonium hydroxide (Fisher Scientific) was added to 0.35 parts of commercially prepared CeO₂ powder (Rhone-Poulenc). The lanthanum and cerium containing mixtures were added together with stirring to form a gel. The resulting gel containing mixture was filtered and the collected solid was calcined in air at 550 deg C for 4 hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. The specific performance data for this example are set forth below in Table 7.

### Reference Example 19

A lanthanum containing composition prepared using the method of Example 5 was ground with a mortar and pestle to form a fine powder. One part of the ground powder was combined with 0.43 parts BaCl₂ powder and further ground using a mortar and pestle to form an intimate mixture. The lanthanum and barium containing mixture was pressed to form chunks. The chunks were calcined at 800 deg C in air for 4 hours. The resulting material was placed in a pure nickel (alloy 200) reactor. The reactor was configured such that ethylene, ethane, HCl, oxygen, and inert (helium and argon mixture) could be fed to the reactor. The specific performance data for this example are set forth below in Table 7.

### Reference Example 20

Dried Grace Davison Grade 57 silica was dried at 120 deg C for 2 hours. A saturated solution of LaCl₃ in water was formed using commercially available hydrated lanthanum chloride. The dried silica was impregnated to the point of incipient wetness with the LaCl₃ solution. The impregnated silica was allowed to air dry for 2 days at ambient temperature. It was further dried at 120 deg C for 1 hour. The resulting material was placed in a pure nickel (alloy 200) reactor. The reactor was configured such that ethylene, ethane, HCl, oxygen, and inert (helium and argon mixture) could be fed to the reactor. The specific performance data for this example are set forth below in Table 7.

### Example 21

A solution of LaCl₃ in water was prepared by dissolving one part of commercially available hydrated lanthanum chloride (purchased from Spectrum Quality Products) in 6.67 parts of deionized water. Rapid addition with stirring of 6 M ammonium hydroxide in water (diluted certified ACS reagent, obtained from Fisher Scientific) caused the formation of a gel. The mixture was centrifuged to collect the solid. Solution was decanted away from the gel and discarded. The gel was resuspended in 12.5 parts of acetone (Fisher Scientific), centrifuged, and the liquid decanted away and discarded. The acetone washing step was repeated 4 additional times using 8.3 parts acetone. The gel was resuspended in 12.5 parts acetone and 1.15 parts of hexamethyldisilizane (purchased from Aldrich Chemical Company) was added and the solution was stirred for one hour. The mixture was centrifuged to collect the gel. The collected gel was allowed to air dry at ambient temperature prior to calcination in air at 550 deg C for four hours. The resulting solid was crushed and sieved. The sieved particles were placed in a pure nickel (alloy 200) reactor. The reactor was configured such that ethylene, ethane, HCl, oxygen, and inert (helium and argon mixture) could be fed to the reactor. The BET surface area is measured to be 58.82 m²/g. The specific performance data for this example are set forth below in Table 7.

### Reference Example 22

A solution of LaCl₃ in water was prepared by dissolving one part of commercially available hydrated lanthanum chloride (Alfa Aesar) and 0.043 parts of commercially available HfCl₄ (purchased from Acros Organics) in 10 parts of deionized water. Rapid addition with stirring of 6 M ammonium hydroxide in water (diluted certified ACS reagent, obtained from Fisher Scientific) caused the formation of a gel. The mixture was centrifuged to collect the solid. Solution was decanted away from the gel and discarded. The collected gel was dried at 80 deg C overnight prior to calcination at 550 deg C for 4 hours. The specific performance data for this example are set forth below in Table 7.

### Reference Example 23

A solution of LaCl₃ in water was prepared by dissolving one part of commercially available hydrated lanthanum chloride (Alfa Aesar) and 0.086 parts of commercially available HfCl₄ (purchased from Acros Organics) in 10 parts of deionized water. Rapid addition with stirring of 6 M ammonium hydroxide in water (diluted certified ACS reagent, obtained from Fisher Scientific) caused the formation of a gel. The mixture was centrifuged to collect the solid. Solution was decanted away from the gel and discarded The collected gel was dried at 80 deg C overnight prior to calcination at 550 deg C for 4 hours. The specific performance data for this example are set forth below in Table 7.

### Reference Example 24

A solution of LaCl₃ in water was prepared by dissolving one part of commercially available hydrated lanthanum chloride (Alfa Aesar) and 0.043 parts of commercially available ZrOCl₂ (purchased from Acros Organics) in 10 parts of deionized water. Rapid addition with stirring of 6 M ammonium hydroxide in water (diluted certified ACS reagent, obtained from Fisher Scientific) caused the formation of a gel. The mixture was centrifuged to collect the solid. Solution was decanted away from the gel and discarded. The gel was resuspended in 6.67 parts deionized water and subsequently centrifuged. The solution was decanted away and discarded. The collected gel was calcined at 550 deg C for 4 hours. The specific performance data for this example are set forth below in Table 7.

### Reference Example 25

A solution of LaCl₃ in water was prepared by dissolving commercially available hydrated lanthanum chloride in deionized water to yield a 2.16 M solution. Commercially produced zirconium oxide (obtained from Engelhard) was dried at 350 deg C overnight. One part of the zirconium oxide was impregnated with 0.4 parts of the LaCl₃ solution. The sample was dried in air at room temperature and then calcined in air at 550 deg C for 4 hours. The resulting solid was crushed and sieved. The sieved particles were placed in a pure nickel (alloy 200) reactor. The reactor was-configured such that ethylene, ethane, HCl, oxygen, and inert (helium and argon mixture) could be fed to the reactor. The specific performance data for this example are set forth below in Table 7.

**Table 7:**

| Rare Earth Compositions with Additional Components | | | | | | | | |
|---|---|---|---|---|---|---|---|---|
| Example | 18 | 19 | 20 | 21 | 22 | 23 | 24 | 25 |
| Feed mole ratios | | | | | | | | |
| C₂H₄ | 3.7 | 3.6 | 3.7 | 3.7 | 3.7 | 3.7 | 3.6 | 3.7 |
| HCl | 2.0 | 2.0 | 2.0 | 2.0 | 2.0 | 2.0 | 2.0 | 2.0 |
| O₂ | 1.0 | 1.0 | 1.0 | 1.0 | 1.0 | 1.0 | 1.0 | 1.0 |
| He+Ar | 0.2 | 0.2 | 0.2 | 0.2 | 0.2 | 0.2 | 0.2 | 0.2 |
| T (°C) | 400 | 401 | 400 | 399 | 401 | 400 | 400 | 401 |
| Space time (s) | 4.8 | 20.3 | 6.7 | 3.6 | 7.9 | 7.8 | 12.8 | 16.7 |

| Fractional conversions (Percent) | | | | | | | | |
|---|---|---|---|---|---|---|---|---|
| C₂H₄ | 18.2 | 11.7 | 14.1 | 24.6 | 18.5 | 16.5 | 18.7 | 15.2 |
| HCl | 34.6 | 22.1 | 24.4 | 57.1 | 40.9 | 38.2 | 35.2 | 21.1 |
| O₂ | 55.6 | 33.2 | 48.0 | 52.0 | 50.3 | 47.4 | 50.9 | 56.4 |

| Selectivities (Percent) | | | | | | | | |
|---|---|---|---|---|---|---|---|---|
| VCM | 64.5 | 54.6 | 53.6 | 56.0 | 76.4 | 71.8 | 73.2 | 55.1 |
| C₂H₄Cl₂ | 11.5 | 15.2 | 10.0 | 31.4 | 9.6 | 12.7 | 5.2 | 7.3 |
| C₂H₅Cl | 5.0 | 10.0 | 7.4 | 2.9 | 4.0 | 4.9 | 4.9 | 12.4 |
| COₓ | 10.8 | 18.6 | 26.6 | 6.0 | 7.6 | 8.8 | 13.6 | 24.1 |

These data show the production of vinyl chloride from ethylene containing streams using lanthanum-based catalysts that contain other elements or are supported.

### Example 26 through Example 31

Example 26 through Example 31 show some of the modifications possible to alter the preparation of useful rare earth compositions.

### Reference Example 26

A solution of LaCl₃ in water was prepared by dissolving one part of commercially available hydrated lanthanum chloride (purchased from Spectrum Quality Products) in 10 parts of deionized water. Rapid addition with stirring of 6 M ammonium hydroxide in water (diluted certified ACS reagent, obtained from Fisher Scientific) caused the formation of a gel. The mixture was centrifuged to collect the solid. Solution was decanted away from the gel and discarded. A saturated solution of 0.61 parts benzyltriethylammonium chloride (purchased from Aldrich Chemical Company) in deionized water was prepared. The solution was added to the gel and stirred. The collected gel was calcined at 550 deg C for 4 hours. The specific performance data for this example are set forth below in Table 8. This example illustrates the use of added ammonium salts to alter the preparation of rare earth compositions.

### Reference Example 27

A solution of LaCl₃ in water was prepared by dissolving one part of commercially available hydrated lanthanum chloride (purchased from Spectrum Quality Products) in 10 parts of deionized water. Rapid addition with stirring of 6 M ammonium hydroxide in water (diluted certified ACS reagent, obtained from Fisher Scientific) caused the formation of a gel. The mixture was centrifuged to collect the solid. One part glacial acetic acid was added to the gel and the gel redissolved. Addition of the solution to 26 pans of acetone caused the formation of a precipitate. The solution was decanted away and the solid was calcined at 550 deg C for 4 hours. The specific performance data for this example are set forth below in Table 8. This example shows the preparation of useful lanthanum compositions by the decomposition of carboxylic acid adducts of chlorine containing rare earth compounds.

### Example 28

A solution of LaCl₃ in water was prepared by dissolving one part of commercially available hydrated lanthanum chloride (purchased from Spectrum Quality Products) in 10 parts of deionized water. Rapid addition with stirring of 6 M ammonium hydroxide in water (diluted certified ACS reagent, obtained from Fisher Scientific) caused the formation of a gel. The mixture was centrifuged to collect the solid. The collected gel was resuspended in 3.33 parts of deionized water. Subsequent addition of 0.0311 parts of phosphoric acid reagent (purchased from Fisher Scientific) produced no visible change in the suspended gel. The mixture was again centrifuged and the solution decanted away from the phosphorus containing gel. The collected gel was calcined for at 550 deg C for 4 hours. The calcined solid had a BET surface area of 33.05 m²/g. The specific performance data for this example are set forth below in Table 8. This example shows the preparation of a rare earth composition also containing phosphorus, as phosphate.

### Example 29

A solution of LaCl₃ in water was prepared by dissolving one part of commercially available hydrated lanthanum chloride (purchased from Acros Organics) in 6.66 parts of deionized water. A solution was formed by mixing 0.95 parts of commercially available DABCO, or 1,4-diazabicyclo[2.2.2]octane, (purchased from ICN Pharmaceuticals) dissolved in 2.6 parts of deionized water. Rapid mixing with stirring of the two solutions caused the formation of a gel. The mixture was centrifuged to collect the solid. The collected gel was resuspended in 6.67 parts of deionized water. The mixture was again centrifuged and the solution decanted away from the gel. The collected gel was calcined for 4 hours at 550 deg C. The calcined solid had a BET surface area of 38.77 m²/g. The specific performance data for this example are set forth below in Table 8. This example shows the utility of an alkyl amine in the preparation of a useful rare earth composition.

### Example 30

A solution of LaCl₃ in water was prepared by dissolving one part of commercially available hydrated lanthanum chloride (purchased from Acros Organics) in 10 parts of deionized water. To this solution, 2.9 parts of commercially available tetramethyl ammonium hydroxide (purchased from Aldrich Chemical Company) was added rapidly and with stirring, causing the formation of a gel. The mixture was centrifuged and the solution decanted away. The collected gel was resuspended in 6.67 parts of deionized water. The mixture was again centrifuged and the solution decanted away from the gel. The collected gel was calcined for 4 hours at 550 deg C. The calcined solid had a BET surface area of 80.35 m²/g. The specific performance data for this example are set forth below in Table 8. This example shows the utility of an alkyl ammonium hydroxide for formation of a useful rare earth composition.

### Example 31

A solution of LaCl₃ in water was prepared by dissolving one part of commercially available hydrated lanthanum chloride (purchased from Avocado Research Chemicals Ltd.) in 6.67 parts of deionized water. To this solution, 1.63 parts of commercially available 5 N NaOH solution (Fisher Scientific) was added rapidly and with stirring, causing the formation of a gel. The mixture was centrifuged and the solution decanted away. The collected gel was calcined for 4 hours at 550 deg C. The calcined solid had a BET surface area of 16.23 m²/g. The specific performance data for this example are set forth below in Table 8. This example shows the utility of non-nitrogen containing bases for the formation of catalytically interesting materials. Although potentially functional the tested materials appear to be inferior to those produced using nitrogen containing bases.

**Table 8:**

| Additional Preparation Methods for Lanthanum Containing Compositions | | | | | | |
|---|---|---|---|---|---|---|
| Example | 26 | 27 | 28 | 29 | 30 | 31 |
| Feed mole ratios | | | | | | |
| C₂H₄ | 3.6 | 3.7 | 3.6 | 3.7 | 3.7 | 3.7 |
| HCl | 2.0 | 2.0 | 2.0 | 2.0 | 2.0 | 2.0 |
| O₂ | 1.0 | 1.0 | 1.0 | 1.0 | 1.0 | 1.0 |
| He+Ar | 0.2 | 0.2 | 0.2 | 0.2 | 0.2 | 0.2 |
| T(°C) | 401 | 400 | 400 | 399 | 400 | 401 |
| Space time (s) | 8.6 | 20.8 | 4.7 | 8.7 | 6.2 | 20.0 |

| Fractional conversions (Percent) | | | | | | |
|---|---|---|---|---|---|---|
| C₂H₄ | 18.8 | 8.7 | 15.6 | 17.4 | 21.0 | 9.3 |
| HCl | 35.8 | 7.7 | 20.0 | 41.5 | 48.4 | 22.3 |
| O₂ | 53.0 | 32.6 | 48.8 | 50.6 | 56.8 | 17.9 |

| Selectivities (Percent) | | | | | | |
|---|---|---|---|---|---|---|
| VCM | 73.4 | 26.0 | 72.1 | 76.8 | 77.6 | 17.5 |
| C₂H₄Cl₂ | 8.7 | 11.9 | 7.1 | 7.3 | 7.8 | 46.2 |
| C₂H₅Cl | 3.5 | 22.7 | 5.6 | 4.2 | 2.9 | 25.6 |
| COₓ | 9.8 | 38.6 | 12.7 | 7.6 | 6.3 | 9.1 |

### Reference Example 32

To further demonstrate the utility of the composition, 1,2-dichloroethane was dehydrochlorinated to yield vinyl chloride by use of the composition as a catalyst. A solution of LaCl₃ in water was prepared by dissolving one part of commercially available hydrated lanthanum chloride (purchased from Avacado Research Chemicals Ltd.) in 6.67 parts of deionized water. Rapid addition with stirring of 6 M ammonium hydroxide in water (diluted certified ACS reagent, obtained from Fisher Scientific) caused the formation of a gel. The mixture was filtered to collect the solid. The collected gel was dried at 120 deg C prior to calcination in air at 550 deg C for four hours. The resulting solid was crushed and sieved. The sieved particles were placed in a pure nickel (alloy 200) reactor. The reactor was configured such that 1,2-dichloroethane and helium could be fed to the reactor. Space time is calculated as the volume of catalyst divided by the flow rate. Feed rates are molar ratios. The composition was heated to 400 deg C and treated with a 1:1:3 HCl:O₂:He mixture for 2 hours prior to the start of operation. The reactor system was operated with ethane and ethylene containing feeds to produce vinyl chloride for 134 hours at a temperature of 400 deg C. At this time the feed composition was altered to contain only He and 1,2-dichloroethane in a 5:1 ratio with the temperature at 400 deg C. Flow was adjusted to yield a 16.0 second space time. Product analysis showed greater than 99.98 percent conversion of 1,2-dichloroethane with the molar vinyl chloride selectivity in excess of 99.11 percent. After 4.6 hours on stream the experiment was terminated. Analysis of the product stream at this time showed conversion of 1,2-dichloroethane to be 99.29 percent with molar selectivity to vinyl chloride of greater than 99.45 percent.

## Claims

1. A catalytically-useful composition comprising a compound of the formula MOCl, wherein M is at least one rare earth element from lanthanum, cerium, neodymium, praseodymium, dysprosium, samarium, yttrium, gadolinium, erbium, ytterbium, holmium, terbium, europium, thulium and lutetium, with the proviso that, when cerium is present, at least one more rare earth element other than cerium is also present, the catalyst being further **characterized** as having a BET surface area of at least 12 m²/g.

2. A catalytically-useful composition comprising a compound of the formula MCl₃ wherein M is at least one rare earth element from lanthanum, cerium, neodymium, praseodymium, dysprosium, samarium, yttrium, gadolinium, erbium, ytterbium, holmium, terbium, europium, thulium and lutetium with the proviso that, when cerium is present; at least one more rare earth element other than cerium is also present, the catalyst being further **characterized** as having a BET surface area of at least 5 m²/g.

3. The composition of any of Claims 1 and 2, wherein M is a mixture of at least two rare earth elements from lanthanum, cerium, neodymium, praseodymium, dysprosium, samarium, yttrium, gadolinium, erbium, ytterbium, holmium, terbium, europium, thulium and lutetium.

4. The composition of any one of Claims 1-3, with the proviso that the composition is substantially free of iron and copper.

5. The composition of any one of Claims 1-4 wherein the BET surface area of the composition is at least 30 m²/g.

6. The composition of Claim 1, as deposited on an inert support.

7. The composition of any one of Claims 1 and 6, wherein the composition is a porous, bulk material.

8. The composition of any one of Claims 1, 6 and 7 wherein the catalyst composition comprises LaOCl.

9. The composition of any one of Claims 1 and 6-8, as prepared by a method comprising the following steps:
(a) preparing a solution of a chloride salt of the rare earth element or elements in a solvent comprising either water, an alcohol, or mixtures thereof;
(b) adding a nitrogen-containing base to cause the formation of a precipitate; and
(c) collecting, drying and calcining the precipitate in order to form the MOCl composition.

10. The composition of Claim 2 wherein the catalyst comprises LaCl₃.

11. The composition of any one of Claims 2 and 10 wherein the composition is prepared by a method comprising the following steps:
(a) preparing a solution of a chloride salt of the rare earth element or elements in a solvent comprising either water, an alcohol, or mixtures thereof;
(b) adding a nitrogen-containing base to cause the formation of a precipitate;
(c) collecting, drying and calcining the precipitate; and
(d) contacting the calcined precipitate with a chlorine source.

12. The composition of Claim 11 wherein the chlorine source is gaseous, and is selected from HCl, Cl₂, and mixtures thereof.

13. A process for producing vinyl chloride from ethylene comprising:
(a) combining reactants including ethylene, an oxygen source, and a chlorine source in a reactor containing a catalyst under conditions sufficient to produce a product stream comprising vinyl chloride and ethylene; the catalyst comprising a composition according to any of claims 1-12, with the proviso that the catalyst is substantially free of iron and copper and with the further proviso that when cerium is present the catalyst further comprises at least one more rare earth element other than cerium.

14. The process of claim 13 further comprising
(b) recycling ethylene from the product stream back for use in Step (a).

15. The process of any of claims 13 or 14, wherein the catalyst further comprises an element selected from alkaline earths, boron, phosphorous, sulfur, silicon, germanium, titanium, zirconium, hafnium, aluminum and combinations thereof.

16. The process of Claim 15, wherein the catalyst employed is **characterized** as being water-soluble after a period of use in the process.

17. The process of any one of Claims 13 to 16, wherein the chlorine source is a gas and comprises at least one of the following: hydrogen chloride, chlorine, a chlorinated hydrocarbon containing labile chlorine, and mixtures thereof.

18. The process of any of Claims 13 to 17, wherein ethane is also combined with the ethylene, oxygen source, and chlorine source in the reactor.

19. The process of Claim 18, wherein the total moles per minute of ethylene entering the reactor is substantially equal to the total moles per minute of ethylene leaving the reactor in the product stream, and further wherein substantially all of the ethylene. leaving the reactor is recycled.

20. The process of Claim 18 in as much it depends on Claim 14, or Claim 17 wherein any ethane present in the product stream is also recycled back for use in Step (a) of the process.

21. The process of any one of Claims 14 to 20 wherein, in Step (b). hydrogen chloride from the product stream is also recycled back for use in Step (a) of the process.

22. The process of any one of Claims 13 to 21 wherein the product stream contains carbon monoxide and carbon monoxide is recycled from the product stream back for use in Step (a) of the process.

23. The process of any one of Claims 13 to 22 wherein the temperature in the reactor is maintained between 350 deg C to 500 deg Celsius.

24. A process for catalytically dehydrochlorinating a feed containing one or more of ethyl chloride, 1,2-dichloroethane, 1,1,2-trichloroethane, using a composition as defined in any one of Claims 1 to 12.

## Patentansprüche

1. Katalytisch geeignete Zusammensetzung, die eine Verbindung der Formel MOCl enthält, worin M wenigstens ein Seltenerdelement aus Lanthan, Cer, Neodym, Praseodym, Dysprosium, Samarium, Yttrium, Gadolinium, Erbium, Ytterbium, Holmium, Terbium, Europium, Thulium und Lutetium ist, unter der Voraussetzung, dass, wenn Cer vorhanden ist, wenigstens ein weiteres Seltenerdelement, das sich von Cer unterscheidet, ebenfalls vorhanden ist, wobei der Katalysator zusätzlich **dadurch gekennzeichnet ist, dass** er eine BET-Oberfläche von wenigstens 12 m²/g aufweist.

2. Katalytisch geeignete Zusammensetzung, enthaltend eine Verbindung der Formel MCl₃, worin M wenigstens ein Seltenerdelement aus Lanthan, Cer, Neodym, Praseodym, Dysprosium, Samarium, Yttrium, Gadolinium, Erbium, Ytterbium, Holmium, Terbium, Europium, Thulium und Lutetium ist, unter der Voraussetzung, dass, wenn Cer vorhanden ist, wenigstens ein weiteres Seltenerdelement, das sich von Cer unterscheidet, ebenfalls vorhanden ist, wobei der Katalysator zusätzlich **dadurch gekennzeichnet ist, dass** er eine BET-Oberfläche von wenigstens 5 m²/g aufweist.

3. Zusammensetzung nach einem der Ansprüche 1 und 2, worin M eine Mischung von wenigstens zwei Seltenerdelementen aus Lanthan, Cer, Neodym, Praseodym, Dysprosium, Samarium, Yttrium, Gadolinium, Erbium, Ytterbium, Holmium, Terbium, Europium, Thulium und Lutetium ist.

4. Zusammensetzung nach einem der Ansprüche 1-3, mit der Bedingung, dass die Zusammensetzung im Wesentlichen frei von Eisen und Kupfer ist.

5. Zusammensetzung nach einem der Ansprüche 1-4, wobei die BET-Oberfläche der Zusammensetzung wenigstens 30 m²/g beträgt.

6. Zusammensetzung nach Anspruch 1 abgeschieden auf einem inerten Träger.

7. Zusammensetzung nach einem der Ansprüche 1 und 6, wobei die Zusammensetzung ein poröses loses Material ist.

8. Zusammensetzung nach einem der Ansprüche 1, 6 und 7, wobei die Katalysatorzusammensetzung LaOCl enthält.

9. Zusammensetzung nach einem der Ansprüche 1 und 6-8, hergestellt durch ein Verfahren, das die folgenden Schritte umfasst:
(a) Herstellung einer Lösung eines Chloridsalzes des Seltenerdelements oder der -elemente in einem Lösungsmittel, das entweder Wasser, einen Alkohol oder Mischungen davon enthält,
(b) Zugeben einer stickstoffhaltigen Base, um die Bildung eines Niederschlags zu bewirken, und
(c) Sammeln, Trocknen und Calcinieren des Niederschlags, um die MOCl-Zusammensetzung auszubilden.

10. Zusammensetzung nach Anspruch 2, wobei der Katalysator LaCl₃ enthält.

11. Zusammensetzung nach einem der Ansprüche 2 und 10, wobei die Zusammensetzung durch ein Verfahren hergestellt ist, das die folgenden Schritte umfasst:
(a) Herstellung einer Lösung eines Chloridsalzes des Seltenerdelements oder der -elemente in einem Lösungsmittel, das entweder Wasser, einen Alkohol oder Mischungen davon enthält,
(b) Zugeben einer stickstoffhaltigen Base, um die Bildung eines Niederschlags zu bewirken, und
(c) Sammeln, Trocknen und Calcinieren des Niederschlags und
(d) In-Kontakt-Bringen des calcinierten Niederschlags mit einer Chlorquelle.

12. Zusammensetzung nach Anspruch 11, wobei die Chlorquelle gasförmig ist und ausgewählt ist aus HCl, Cl₂ und Mischungen davon.

13. Verfahren zur Herstellung von Vinylchlorid aus Ethylen, umfassend:
(a) Vereinigen der Reaktanten, einschließlich Ethylen, einer Sauerstoffquelle und einer Chlorquelle, in einem Reaktor, der einen Katalysator enthält, unter Bedingungen, die ausreichen, um einen Produktstrom, der Vinylchlorid und Ethylen enthält, zu erzeugen, wobei der Katalysator eine Zusammensetzung nach einem der Ansprüche 1-12 enthält, unter der Voraussetzung, dass der Katalysator im Wesentlichen frei von Eisen und Kupfer ist, und unter der weiteren Voraussetzung, dass, wenn Cer vorhanden ist, der Katalysator zusätzlich wenigstens ein weiteres Seltenerdelement enthält, das sich von Cer unterscheidet.

14. Verfahren nach Anspruch 13, zusätzlich umfassend:
(b) Zurückführen von Ethylen aus dem Produktstrom zur Verwendung in Schritt (a).

15. Verfahren nach einem der Ansprüche 13 oder 14, wobei der Katalysator weiterhin ein Element, ausgewählt aus Erdalkalimetallen, Bor, Phosphor, Schwefel, Silicium, Germanium, Titan, Zirconium, Hafnium, Aluminium und Kombinationen davon, enthält.

16. Verfahren nach Anspruch 15, wobei der eingesetzte Katalysator **dadurch gekennzeichnet, dass** er nach einer Nutzungsdauer in dem Verfahren wasserlöslich ist.

17. Verfahren nach einem der Ansprüche 13 bis 16, wobei die Chlorquelle ein Gas ist und wenigstens eines der Folgenden umfasst: Chlorwasserstoff, Chlor, einen chlorierten Kohlenwasserstoff, der labiles Chlor enthält, und Mischungen davon.

18. Verfahren nach einem der Ansprüche 13 bis 17, wobei Ethan auch mit Ethylen, der Sauerstoffquelle und der Chlorquelle in dem Reaktor vereinigt wird.

19. Verfahren nach Anspruch 18, wobei die Gesamtmolzahl pro Minute an Ethylen, die in den Reaktor eintritt, im Wesentlichen gleich zu der Gesamtmolzahl pro Minute an Ethylen ist, die den Reaktor im Produktstrom verlässt, und wobei weiterhin im Wesentlichen das gesamte Ethylen, das den Reaktor verlässt, zurückgeführt wird.

20. Verfahren nach Anspruch 18, sofern dieser auf Anspruch 14 oder Anspruch 17 zurückbezogen ist, wobei jegliches Ethan, das in dem Produktstrom vorhanden ist, ebenfalls zur Verwendung in Schritt (a) des Verfahren zurückgeführt wird.

21. Verfahren nach einem der Ansprüche 14 bis 20, wobei in Schritt (b) Chlorwasserstoff aus dem Produktstrom ebenfalls zur Verwendung in Schritt (a) des Verfahrens zurückgeführt wird.

22. Verfahren nach einem der Ansprüche 13 bis 21, wobei der Produktstrom Kohlenmonoxid enthält und Kohlenmonoxid aus dem Produktstrom zur Verwendung in Schritt (a) des Verfahrens zurückgeführt wird.

23. Verfahren nach einem der Ansprüche 13 bis 22, wobei die Temperatur in dem Reaktor zwischen 350°C bis 500°C gehalten wird.

24. Verfahren zur katalytischen Dehydrochlorierung eines Zuführstroms, enthaltend ein oder mehrere von Ethylchlorid, 1,2-Dichlorethan, 1,1,2-Trichlorethan, unter Verwendung einer Zusammensetzung wie in einem der Ansprüche 1 bis 12 definiert.

## Revendications

1. Composition catalytiquement utile comprenant un composé de formule MOCl, dans laquelle M est au moins un lanthanide parmi le lanthane, le cérium, le néodyme, le praséodyme, le dysprosium, le samarium, l'yttrium, le gadolinium, l'erbium, l'ytterbium, l'holmium, le terbium, l'europium, le thulium et le lutétium, à la condition que, quand le cérium est présent, au moins un lanthanide supplémentaire autre que le cérium soit également présent, le catalyseur étant en outre **caractérisé** comme ayant une surface active BET d'au moins 12 m²/g.

2. Composition catalytiquement utile comprenant un composé de formule MCl₃, dans laquelle M est au moins un lanthanide parmi le lanthane, le cérium, le néodyme, le praséodyme, le dysprosium, le samarium, l'yttrium, le gadolinium, l'erbium, l'ytterbium, l'holmium, le terbium, l'europium, le thulium et le lutétium,- à la condition que, quand le cérium est présent, au moins un lanthanide supplémentaire autre que le cérium est également présent, le catalyseur étant en outre **caractérisé** comme ayant une surface active BET d'au moins 5 m²/g.

3. Composition selon l'une quelconque des revendications 1 et 2, dans laquelle M est un mélange d'au moins deux lanthanides parmi le lanthane, le cérium, le néodyme, le praséodyme, le dysprosium, le samarium, l'yttrium, le gadolinium, l'erbium, l'ytterbium, l'holmium, le terbium, l'europium, le thulium et le lutétium.

4. Composition selon l'une quelconque des revendications 1 à 3, à la condition que la composition soit essentiellement dépourvue de fer et de cuivre.

5. Composition selon l'une quelconque des revendications 1 à 4, dans laquelle la surface active BET de la composition est d'au moins 30 m²/g.

6. Composition selon la revendication 1, déposée sur un support inerte.

7. Composition selon l'une quelconque des revendications 1 et 6, dans laquelle la composition est un matériau en vrac, poreux.

8. Composition selon l'une quelconque des revendications 1, 6 et 7, dans laquelle la composition de catalyseur comprend du LaOCl.

9. Composition selon l'une quelconque des revendications 1 et 6 à 8, préparée par un procédé comprenant les étapes suivantes consistant à :
(a) préparer une solution d'un sel de chlorure du ou des lanthanides dans un solvant comprenant de l'eau, un alcool, ou un de leurs mélanges ;
(b) ajouter une base contenant de l'azote pour entraîner la formation d'un précipité ; et
(c) recueillir, sécher et calciner le précipité pour former la composition de MOCl.

10. Composition selon la revendication 2, dans laquelle le catalyseur comprend du LaCl₃.

11. Composition selon l'une quelconque des revendications 2 et 10, dans laquelle la composition est préparée par un procédé comprenant les étapes suivantes consistant à :
(a) préparer une solution d'un sel de chlorure du ou des lanthanides dans un solvant comprenant de l'eau, un alcool ou un de leurs mélanges ;
(b) ajouter une base contenant de l'azote pour entraîner la formation d'un précipité ;
(c) recueillir, sécher et calciner le précipité ; et
(d) mettre en contact le précipité calciné avec une source de chlore.

12. Composition selon la revendication 11, dans laquelle la source de chlore est gazeuse, et est choisie parmi HCl, Cl₂, et leurs mélanges.

13. Procédé de production de chlorure de vinyle à partir d'éthylène comprenant :
(a) la combinaison de réactifs comprenant l'éthylène, une source d'oxygène, et une source de chlore dans un réacteur contenant un catalyseur dans des conditions suffisantes pour produire un courant de produit comprenant du chlorure de vinyle et de l'éthylène ; le catalyseur comprenant une composition selon l'une quelconque des revendications 1 à 12, à la condition que le catalyseur soit essentiellement sans fer et cuivre et à l'autre condition que, lorsque le cérium est présent, le catalyseur comprenne au moins un lanthanide supplémentaire autre que le cérium.

14. Procédé selon la revendication 13, comprenant en outre
(b) le recyclage de l'éthylène du courant de produit en retour pour une utilisation dans l'étape (a).

15. Procédé selon l'une quelconque des revendications 13 ou 14, dans lequel le catalyseur comprend en outre un élément choisi parmi les alcalino-terreux, le bore, le phosphore, le soufre, le silicium, le germanium, le titane, le zirconium, l'hafnium, l'aluminium et leurs combinaisons.

16. Procédé selon la revendication 15, dans lequel le catalyseur employé est **caractérisé** comme étant hydrosoluble après une période d'utilisation dans le procédé.

17. Procédé selon l'une quelconque des revendications 13 à 16, dans lequel la source de chlore est un gaz et comprend au moins un élément parmi ce qui suit : le chlorure d'hydrogène, le chlore, un hydrocarbure chloré contenant un chlore labile, et leurs mélanges.

18. Procédé selon l'une quelconque des revendications 13 à 17, dans lequel de l'éthane est aussi combiné avec l'éthylène, la source d'oxygène, et la source de chlore dans le réacteur.

19. Procédé selon la revendication 18, dans lequel le nombre total de moles d'éthylène entrant par minute dans le réacteur est essentiellement égal au nombre total de moles d'éthylène quittant par minute le réacteur dans le courant de produit, et en outre dans lequel essentiellement l'ensemble de l'éthylène quittant le réacteur est recyclé.

20. Procédé selon la revendication 18 pour autant qu'il dépend de la revendication 14, ou de la revendication 17, dans lequel tout éthane présent dans le courant de produit est aussi recyclé pour une nouvelle utilisation dans l'étape (a) du procédé.

21. Procédé selon l'une quelconque des revendications 14 à 20, dans lequel, dans l'étape (b), le chlorure d'hydrogène du courant de produit est aussi recyclé pour une nouvelle utilisation dans l'étape (a) du procédé.

22. Procédé selon l'une quelconque des revendications 13 à 21, dans lequel le courant de produit contient du monoxyde de carbone et le monoxyde de carbone est recyclé du courant de produit pour une nouvelle utilisation dans l'étape (a) du procédé.

23. Procédé selon l'une quelconque des revendications 13 à 22, dans lequel la température dans le réacteur est maintenue entre 350 et 500 degrés Celsius.

24. Procédé pour dé-chlorhydrater catalytiquement une alimentation contenant un élément ou plusieurs parmi le chlorure d'éthyle, le 1,2-dichloroéthane, le 1,1,2-trichloroéthane, en utilisant une composition telle que définie dans l'une quelconque des revendications 1 à 12.
